# EUROPEAN PATENT SPECIFICATION

(11) **EP 3 530 308 B1**
(45) Date of publication and mention of the grant of the patent: **12.05.2021**
(21) Application number: 19158119.8
(22) Date of filing: 19.02.2019
(51) Int. Cl.: A61M 16/10, A61M 16/00, A61M 16/12, A61M 16/08

(54) **EMERGENCY OXYGEN DELIVERY DEVICE**
NOTSAUERSTOFFZUFUHRVORRICHTUNG
DISPOSITIF DE DISTRIBUTION D'OXYGÈNE D'URGENCE

(30) Priority: 23.02.2018 US 201862634335 P
(43) Date of publication of application: 28.08.2019
(73) Proprietor: L'AIR LIQUIDE, SOCIETE ANONYME POUR L'ETUDE ET L'EXPLOITATION DES PROCEDES GEORGES CLAUDE, 75007 Paris (FR)
(72) Inventor: BOULANGER, Thierry, Media, Pennsylvania 19063 (US)
(74) Representative: Air Liquide

(56) References cited:
- EP-A1- 3 019 228
- WO-A1-2004/064907
- GB-A- 806 471
- US-A- 4 573 462
- US-A- 4 883 051

## Description

The present invention concerns a gas delivery device or apparatus for delivering an oxygen gas flow to a person in need thereof, especially in an emergency situation.

Oxygen is commonly administrated by inhalation for correcting a hypoxemia, e.g. an oxyhemoglobin saturation below normal ranges (e.g., < 85%) in a patient. Actually, oxygen is widely used for treating various patient populations, e.g. neonates to adult, as well as various medical conditions, e.g. pulmonary deficiencies, such as Acute Respiratory Distress Syndrome (ARDS), Chronic Obstructive Pulmonary Disease (COPD) or post-operative recovery only.

In a hospital, oxygen is usually delivered through wall outlets or plugs fluidly connected to an oxygen source(s), for instance a large-volume vessel of liquid oxygen, and preferably a backup system, coupled to a vaporizer so as to ensure a continuous supply of gaseous oxygen to the patients of the hospital.

Large-volume oxygen vessels are not available in emergency units, such as ambulances or the like, while numerous emergency situations require oxygen for treating the persons involved, e.g., car crash, cardiac arrest situation, pulmonary deficiencies.... In those emergency situations, the medical staff, such as doctors, nurses or the like, only relies on gas cylinders having a limited size, typically from 2 to 5 liter cylinders filled with medical grade compressed oxygen.

The oxygen flow delivered by such a cylinder is set by the medical staff depending on the patient to be treated. For instance, it is usually to deliver a flow of 3L/min of oxygen to an infant and of 6 L/min to an adult. A pressure/flowrate regulator or valve arranged on the cylinder is usually used for setting the desired oxygen flowrate.

However, if such regulators are highly effective, they are mechanically complex and rather expensive due to the presence of a calibrated-orifice plate mechanism for setting the flowrate arranged inside the regulator body or of other specific parts. GB 806471 A, US 4883051 A, US 4573462 A, EP 3019228 A1, WO 2004/064907 A1 disclose examples of breathing systems.

A goal of the present invention is to propose a simpler and less expensive gas delivery device that can be arranged on a gas cylinder. The invention is defined by the appended claim 1. The embodiments disclosed herein are considered as examples unless defined as embodiments of the invention.

Another goal of the present disclosure is to propose a gas delivery device that allows a delivery of steady flows of oxygen, preferably of between about 3 to about 6 L/min of oxygen, for a given period of time, preferably of at least 20 minutes.

Another goal of the present disclosure is to propose a gas delivery device that is useable in emergency situations for treating people in need of oxygen gas.

Another goal of the present disclosure is to propose a gas delivery device that does not include any calibrated-orifice plate mechanism as it is the case in pressure/flowrate regulators of the prior art.

Another goal of the present disclosure is to propose a gas delivery device that is suitable for delivering oxygen gas to adults as well as to children in need thereof.

A solution of the present invention comprises a gas delivery device or apparatus comprising:
- a main chamber,
- an injection nozzle comprising an inner gas passage in fluid communication with the main chamber, said inner gas passage comprising an inlet diameter and an outlet diameter, said inlet diameter being greater than the outlet diameter,
- a downstream chamber in fluid communication with the inner gas passage of the injection nozzle,
- an inflatable bag in fluid communication with the downstream chamber,
- a second chamber fluidly communicating with the main chamber and further with the inflatable bag, and
- a gas-flow control device arranged between the main chamber and the second chamber for controlling the passage of gas from the main chamber to the second chamber.

Depending on the embodiment, a gas delivery device or apparatus according to the present disclosure can comprise one or several of the following features:
- the inner gas passage has a conical or tronconical shape, i.e., structure.
- the inner gas passage comprises or forms a Venturi system.
- the gas flow control device comprises a flexible membrane.
- the gas flow control device further comprises an elastic element cooperating with the flexible membrane.
- the flexible membrane is made of silicon
- the elastic element comprises a spring element.
- the inflatable bag is made of a flexible material, such as polyethylene (PE) or a combination of polyethylene and one or several aluminum foils.

The disclosure also concerns a gas cylinder comprising a gas delivery device according to the present disclosure, such as an oxygen cylinder, preferably a oxygen cartridge.

The present disclosure will be explained in more details in the following illustrative description of an embodiment of a gas delivery device according to the present disclosure mounted on an oxygen gas cylinder, which is made in references to the accompanying drawings among them:
- Fig. 1 and 2 show two embodiments of an oxygen source useable with a gas delivery device according to the present invention,
- Fig. 3 shows an embodiment of a gas delivery device according to the present invention,
- Fig. 4A to 4E show a subset of the gas delivery device of Fig. 3,
- Fig. 5 to 9 show the gas delivery device of Fig. 3 in use, when mounted on the oxygen source of Fig. 1, and
- Fig. 10 represents the distribution of gas flows in the gas delivery device according to the present invention, during use.

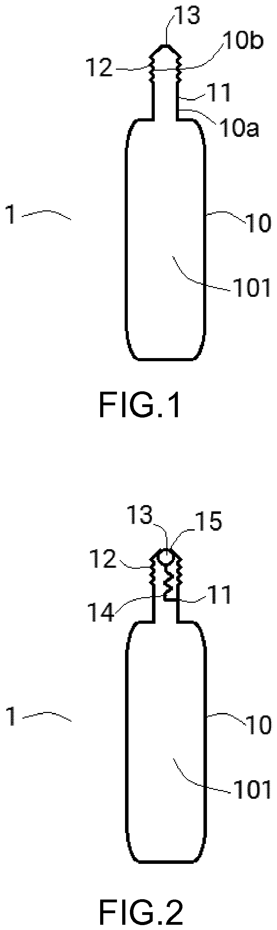

Fig.1 represents an embodiment of an oxygen source 1, preferably an oxygen cylinder, useable in the frame of the present invention. The oxygen source 1 comprises a disposable cartridge 10, for instance made of an aluminum alloy, comprising, at its upper end 10a, a cartridge outlet 11 sealed by a closing cap 13 so that the gas compressed in said cartridge 10 cannot flow out of it.

An outer threaded portion 12 is arranged in the peripheral outer wall 10b of the cartridge's upper end 10a for allowing securing a gas delivery device according to the present invention to the oxygen source 1.

The internal volume 101 of cartridge 10 is sized so as to contain, in this embodiment, about 0.5L of compressed oxygen at a pressure of 200 bars, preferably medical grade oxygen, thereby ensuring a delivery of about 120 L of oxygen, e.g. 6L/min for 20 minutes. Of course, the cartridge 10 could be sized for containing other volumes of compressed oxygen.

Fig. 2 shows an alternative embodiment of the oxygen source 1 similar to the one of claim 1, except it further comprises a ball-system valve 13, 14 comprising a ball 13, preferably a metallic ball, loaded by a spring 14, for normally closing the cylinder outlet orifice 15. Such a ball-system valve 13, 14 can be used in lieu of the closing cap 13 of Figure 1.

Cartridges 10 according to Figures 1 and 2 will not be further detailed as they are well-known and available on the market.

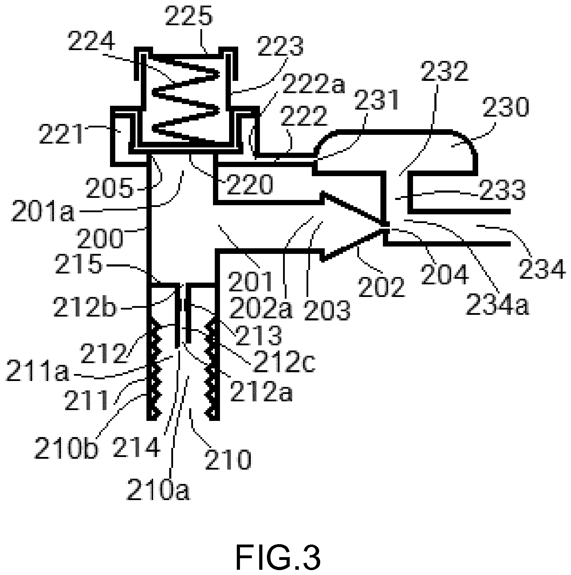

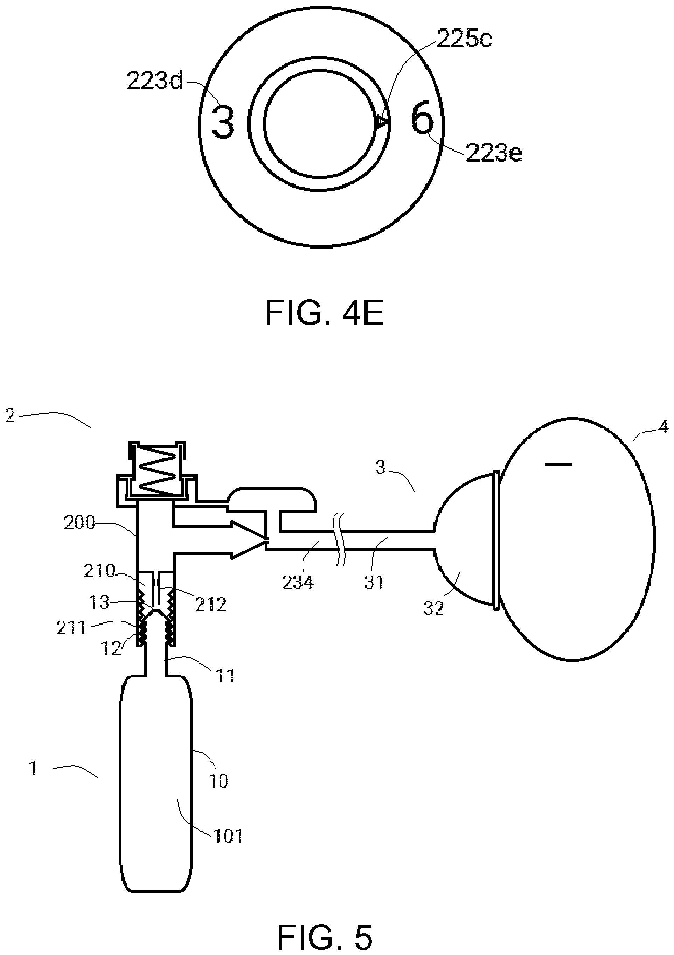

Figure 3 shows an embodiment of a gas delivery device 2 according to the present invention that is useable, especially in emergency situations, for delivering a flow of oxygen when mounted on an oxygen source 1 (see Fig. 5), such as an oxygen cartridge 10, as shown in Figures 1 and 2, or to any other suitable oxygen cylinder.

Said gas delivery device 2 comprises a hollow connecting portion 211a comprising an axial passage 210a for the gas having an inlet 210. The connecting portion 211a further comprises an external armature 200 that surrounds the peripheral outer wall 10b of the cartridge's upper end 10a, when the gas delivery device 2 is fixed to the cartridge's upper end 10a, as shown in Figures 5, 6 and 8.

The inner wall 210b of the axial passage 210a comprises an inner threaded portion 211 that is complementary to the outer threaded portion 12 arranged on the oxygen cartridge 10 so that both threaded portions 211, 12 can cooperate and be coupled together for securing the gas delivery device 2 to the oxygen source 1.

A hollow elongated stem 212 is axially arranged in the axial passage 210a and is securely attached to the inner wall 210b of the axial passage 210a of the armature 200 by a prolongation wall 215. The hollow elongated stem 212 ensures a fluidic communication between the inlet 210 and a main chamber 201 via a first stem port 212a and second stem port 212b located at the opposite ends of the stem inner passage 212c traversing the hollow elongated stem 212.

The stem inner passage 212c further comprises a flow restriction, preferably a calibrated orifice 213 for limiting the flow of oxygen circulating into the stem inner passage 212c of the hollow elongated stem 212, in the direction of the main chamber 201. For instance, the diameter of the calibrated orifice 213 is of about 0.025 mm; of course, it could have a greater or lesser diameter.

Main chamber 201 is in fluid communication with an inner gas passage 202a of an injection nozzle 202 arranged downstream of said main chamber 201. In other words, the main chamber 201 is prolonged by, at one of its end, an injection nozzle 202 having an inner conical or tronconical shape or structure, so that the gas exiting the main chamber 201 enters the inner gas passage 202a of the injection nozzle 202.

Said inner gas passage 202a of conical or tronconical shape comprises an inlet diameter 203 located on the side of the main chamber 201, and an outlet diameter 204 located on the other side, the inlet diameter 203 being greater than the outlet diameter 204, preferably the inlet diameter 203 is at least 10 times the outlet diameter 204. For instance, the inlet diameter 203 can be of between 10 and 20 mm, whereas the outlet diameter 204 can be of between 0.3 and 0.4 mm.

The outlet diameter 204 of the injection nozzle 202 is in fluid communication with a downstream chamber 233, such as the lumen 234a of a conduit 234, arranged downstream of the injection nozzle 202 so that gas delivered by the injection nozzle 202 is recovered by said downstream chamber 233, preferably a conduit 234, and circulates into the lumen 234a of said conduit 234.

The particular structure of the injection nozzle 202 forms a Venturi system. The diameter decrease from inlet diameter 203 to outlet diameter 204 leads to an increase of the gas velocity traversing the injection nozzle 202 as well as a localized depression (i.e. a negative pressure) downstream of the outlet orifice 204, e.g. in downstream chamber 233. Such a negative pressure ensures an additional gas suction so that the flow of gas circulating in the downstream chamber 233, especially in the lumen 234a of conduit 234, is greater that the flow circulating in injection nozzle 202.

Said downstream chamber 233, especially in the lumen 234a of conduit 234, is further in fluid communication with the bag outlet 232 of an inflatable bag 230. Preferably, said inflatable bag 230 is made of a flexible material, such as polyethylene (PE) or a combination of polyethylene and aluminum foils.

Further, said inflatable bag 230 also comprises a bag inlet 231. A connection conduct 222 fluidly links the bag inlet 231 to the exit port 222a of a second chamber 221 that is fluidly communicating with the main chamber 201, via outlet orifice 201a, as shown in Fig. 3. Thanks to the connection conduct 222, the second chamber 221 is fluidly communicating with the inflatable bag 230 as explained below.

Further, a gas-flow control device 220, 224 is arranged between the main chamber 201 and the second chamber 221 for controlling the passage of gas from the main chamber 201 to the second chamber 221. In one embodiment, the gas-flow control device 220, 224 comprises a flexible membrane 220 and an elastic element 224, such as a spring element, for instance a metallic spring, cooperating with the flexible membrane 220.

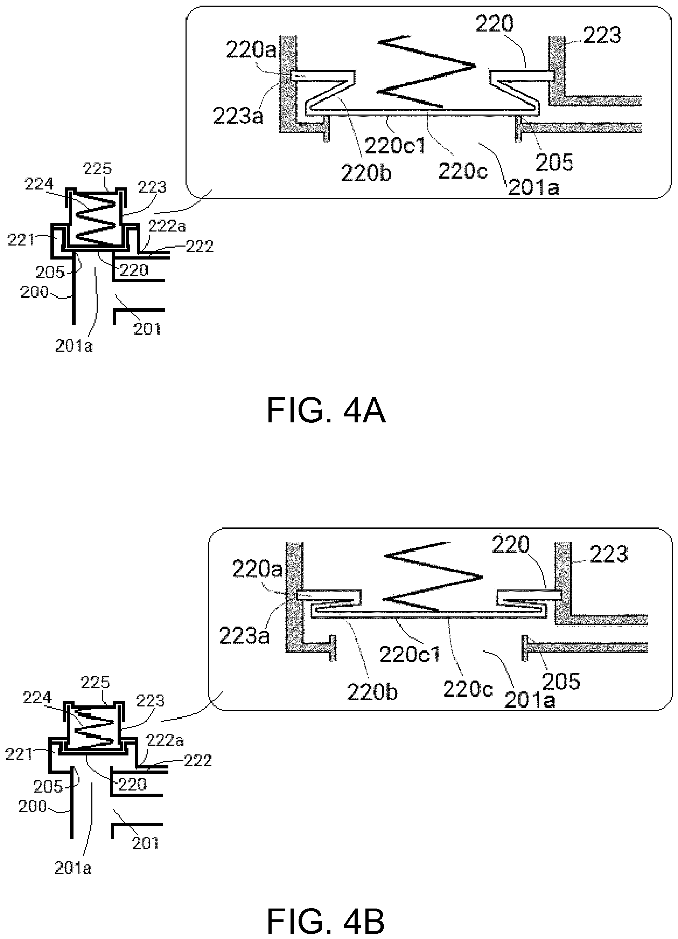

The gas-flow control device 220, 224 is shown in Figures 4A-4E.

As shown in Fig. 4A, membrane 220 is normally tightly closing the outlet orifice 201a located between main chamber 201 and second chamber 221 by resting via its lower surface 220c, on the peripheral edges 205 of the armature 200 that form an annular seat, thereby prohibiting any gas passage from the main chamber 201 to the second chamber 221. Membrane 220 is held in such a "closing position" thanks to the force applied by elastic element 224 that is embedded into a spring armature 223 fixed to the walls of the second chamber 221, and maintained therein by a closing cap 225.

The elastic element 224 is normally pushing against the upper surface 220c of the membrane 220, i.e. in the direction of the main chamber 201. The membrane 220 is deformable. It comprises a lip portion 220a which is securely attached to a grove 223a arranged in the spring armature 223, and further a deformable portion 220b.

Whenever the gas force acting on the lower surface 220c1 of membrane 220, which is related to the pressure in chamber 201, is greater than the force developed by the spring element 224, the deformable portion 220b of membrane 220 executes an upward movement which in turns lifts its upper surface 220c, as shown in Fig. 4B.

As a result, a fluidic pathway is created between chambers 201 and 221, through outlet orifice 201a, and consequently with the lumen of the connection conduct 222, thereby allowing gas to circulate successively in main chamber 201, second chamber 221, connection conduct 222 and inflatable bag 230.

The spring element 224 is designed so that, in the configuration of Fig. 4B, the opening pressure, e.g. pressure in chamber 201 which is sufficient to push the surface 220c of membrane 220 upwardly, is set to be of about 500 mbar.

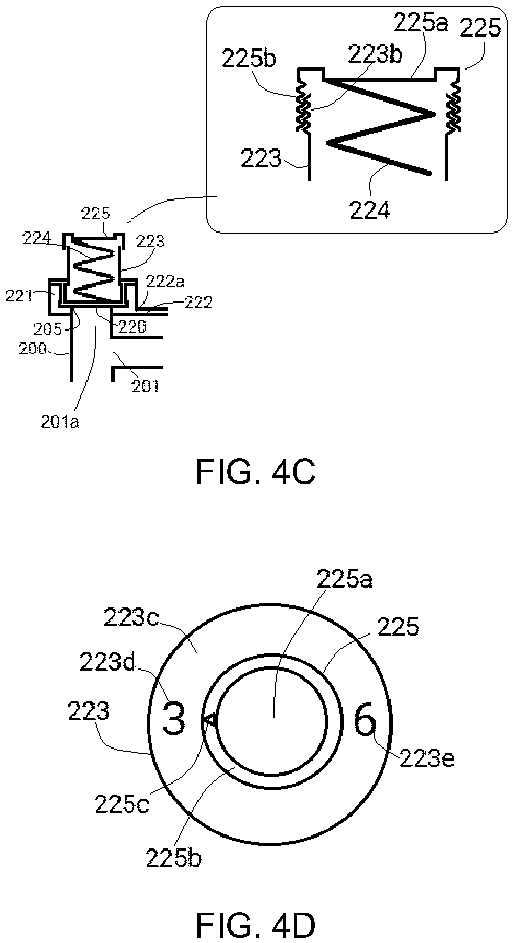

Said opening pressure depends on the specifications of the spring element 224 and its initial compression state defined by the position of roof plate 225a of closing cap 225 as shown in Fig. 4C. Indeed, the threaded portion 225b of closing cap 225 and the complementary threaded portion 223b of spring armature 223 allow roof plate 225a moving downwardly or upwardly, when said closing cap 225 is more or less screwed onto spring armature 223 by a user.

These up or down motions will set a length of spring 224 as defined by the distance between roof plate 225a and upper surface 220c of membrane 220, that sets the opening pressure.

As an example, Fig. 4D, which is a view from above of armature 223 and cap 225, includes guidance to the operator to set two opening pressures. Thus, the upper surface 225b of closing cap 225 embeds a marking, such as an arrow 225c or any other marking, which can be either embossed or printed in said upper surface 225b, whereas upper surface 223c of armature 223 shows, in the present embodiment, two positions, likewise either embossed or printed, namely position "3" 223d and position "6" 223e, that are diametrically opposed.

By turning/screwing closing cap 225 so as to make arrow 225c coincide with position "3" 223d as shown in Fig. 4D or with position "6" 223e as shown in Fig. 4E, the user is able to set a desired opening pressure corresponding to either positions, for instance a pressure of about 200mb in position "3" and of about "500mb" in position "6".

Position "6" is taken below as an example in connection with Fig. 5 to 10 that show successive steps to be taken for securing a gas delivery device 2 according to the present invention to an oxygen source 1.

Fig. 5 represents a first step of pairing/connecting the oxygen source 1 of Figure 1 to the gas delivery device 2 of Figure 3. In Fig. 5, a patient 4 is connected via breathing pathway 3 to the gas delivery device 2 of Fig. 3, according to the present invention. The breathing pathway 3 comprises a gas conduct 31, such as a flexible hose, connected to conduit 234 of the delivery device 2 and ended by an interface 32, such as a nasal cannula or an oro-nasal mask as shown. As one can see, the oxygen source 1, e.g. cartridge 10, is inserted into inlet 210 of the gas delivery device 2, and threaded portion 211 of armature 200 and threaded portion 12 of outlet 11 are put into contact and are screwed together for securing the gas delivery device 2 on oxygen source 1. This can be done by turning the canister 10 clockwise. In this initial step, the cap 13 of outlet 11 does not yet enter into contact with stem 212 so that the gas contained in internal volume 101 of cartridge 10 cannot escape.

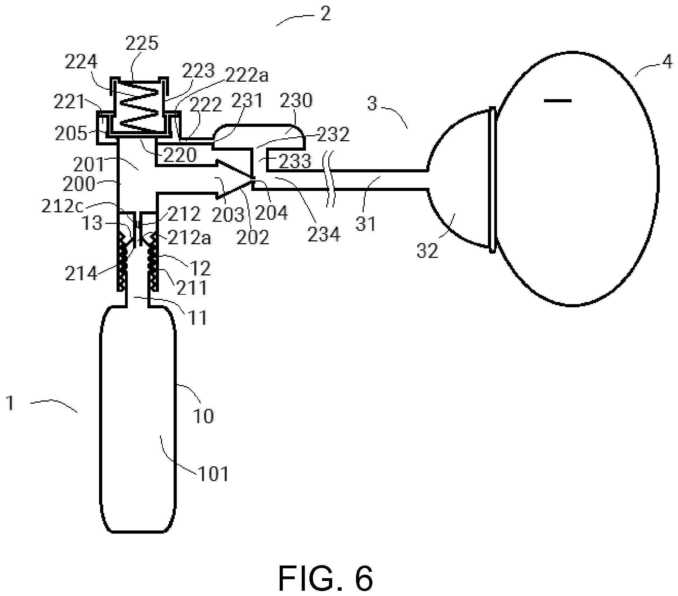

Fig. 6 represents a second step of the insertion of the oxygen source 1 by turning canister 10 further, clockwise. At this point, a contact between elongated stem 212 and cap 13 closing the outlet 11 of cartridge 10 has already taken place.

Actually, the tip 214 of stem 212, i.e. its free end 212a, has punctured/pierced the cap 13 of cartridge 10 so that a fluidic connection is established between the internal volume 101 of canister 10 and main chamber 201 as the gas can circulates in the stem inner passage 212c traversing the hollow elongated stem 212, i.e. in the lumen of elongated stem 212, in the direction of the main chamber 201. The complementary threaded portions 211 and 12 securely maintain the canister 10 in position thereby preventing any damages that might arise with the compressed oxygen source 1.

As the puncture of cap 13 just happened (at t=0 sec) in Fig. 6, the elements located downstream of stem 212 are still in their native configuration (i.e., as in Fig. 5), namely main chamber 201 is at ambient conditions, membrane 220 tightly closes the fluidic pathway between said main chamber 201 and second chamber 221, and no gaseous flow is traversing the injection nozzle 202.

Calibrated orifice 213, arranged in stem 212, acts as a flow limiter to prevent an excessive amount of gas flowing out of canister 10.

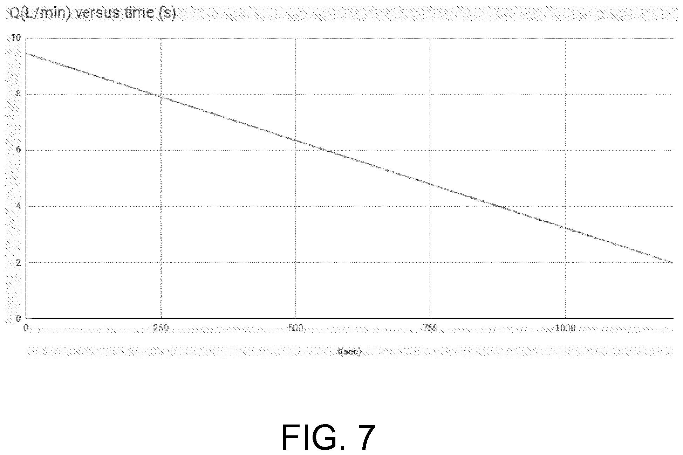

Based on the dimensions of orifice 213 and assuming an internal volume 101 of canister 10 with an initial filling pressure of 200 bars, the resulting flow coming out of outlet 11 and circulating into stem 212 to fill the main chamber 201, can have an initial value of about 10L/min (flowrate), right after tip 214 of stem 212 punctured cap 13, to progressively decrease over the time, for reaching 2L/min after about 20 min of operation, as shown in Figure 7.

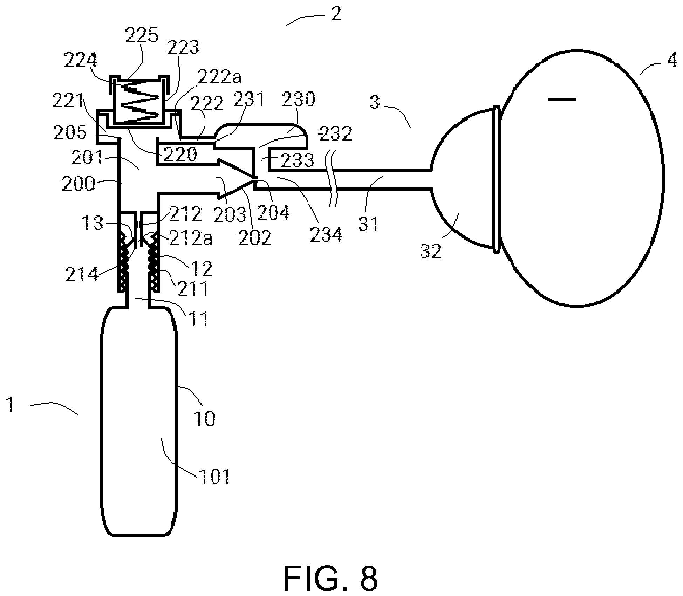

Once a flow of oxygen leaves the oxygen source 1, a pressure increase occurs in main chamber 201 as the gaseous flow traverses stem 212. This pressure rise mainly depends on the design of injection nozzle 202, especially its outlet diameter 204 as it creates an additional resistance to the flow coming from the internal volume 101 of canister 10. As an example, a diameter of about 0.035 mm of the outlet orifice 204 generates a back pressure of 500 mb if a flow of 1L/min passes through it. This is precisely the opening pressure of membrane 220 as described above, namely the position "6" of cap 225. In other words, if the flow coming from the oxygen source is greater than 1 L/min, a fluidic connection between main and second chambers 201, 221 is created, as shown in Fig. 8 and the pressure within main chamber 201 remains steady at the opening pressure of 500 mb.

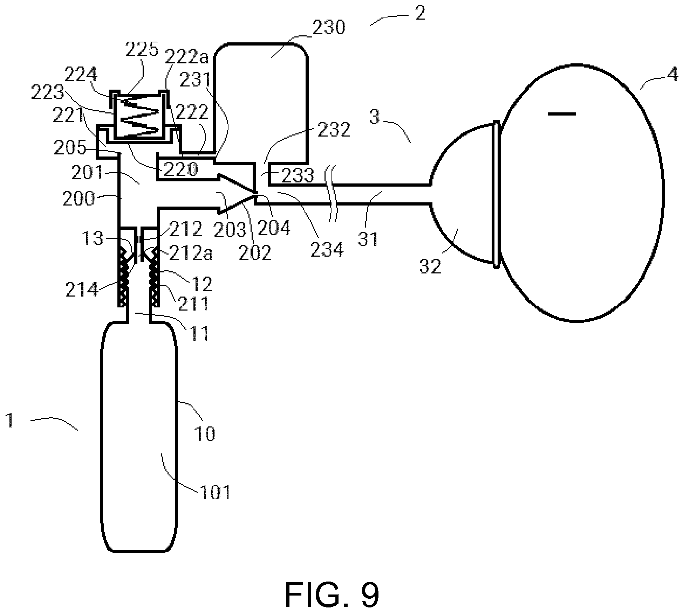

Furthermore, as liaison conduct 222 acts as a fluidic bridge between inlet 222a, in contact with second chamber 221, and inflatable bag 230 via inlet 231, a fluidic connection is made between main chamber 201 and bag 230. Consequently, as long as the flow coming from the oxygen source 1 is greater than 1L/min, the flow in excess is redirected from main chamber 201 into inflatable bag 230 that starts to inflate, as shown in Figure 9.

The pressure within main chamber 201 is of 500 mb, whereas the inflatable bag 230 collects the gas excess coming from oxygen source 1. The pressure (i.e. 500mb) at the inlet 203 of the injection nozzle 202 generates a flowrate of about 1 L/min that is ejected through outlet 204. Due to the fluid acceleration occurring in nozzle 202, a depression occurs in chamber 233 that draws gas out of inflatable bag 230 via outlet 232. The design of chamber 233 is such that for an incoming flow of 1 L/min in the nozzle 202 (e.g. 500mb at inlet 203), a 6-time increase is noticed in conduct 234. In other words, the depression created in chamber 233 draws an additional 5 L/min of gas that is combined with the incoming flow of 1 L/min to generate a total flow of 6 L/min, reaching patient 4 via the breathing pathway 3.

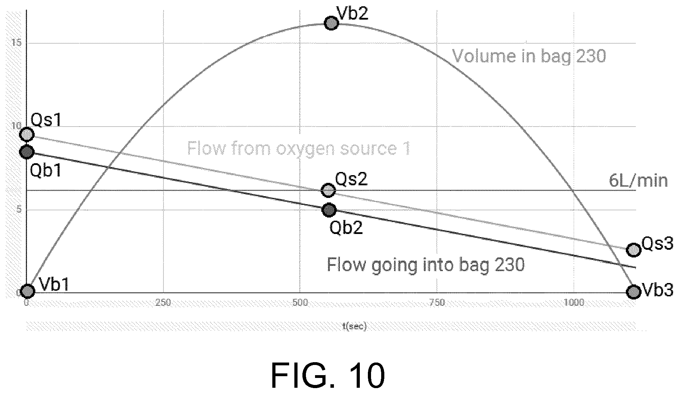

As can be seen in Figure 10, the flow coming from oxygen source 1 is of about 10 L/min (Qs1) at the onset of the delivery (t=0) to about 2 L/min (Qs3) after 20 min of operation (t=1200s).

This means that enough flow is generated over the course of the operation to have 1 L/min crossing the injection nozzle 202 and therefore reaches the opening pressure of 500 mb in main chamber 201. As a result, an initial flow Qb1 of 9L/min (10 L/min - 1L/min) fills the inflatable bag 230 as above explained.

As the design of the Venturi made of injection nozzle 202 and second chamber 233 draws 5L/min from bag 230 for such operating conditions, a remaining 4L/min flow (i.e. 9L/min - 5L/min) is in excess and stays in bag 230. This is the inflation phase. As long as the flow coming from the oxygen source 1 is greater than 6 L/min, i.e. between Qs1 and Qs2, the bag 230 inflates to start from an initial value of 0 L (Vb1) to a maximum value of about 17 L (Vb2) after 10 minutes (t=600s). During these first 10 minutes, the flow traveling into conduit 234 and reaching patient 4 via breathing pathway 3 equals 6L/min.

Once the flow coming from oxygen source 1 is less than 6L/min (Qs2), the flow going into bag 230 (Qb2) becomes less than the flow of 5L/min drawn into chamber 233 via the Venturi effect. The accumulated volume in bag 230 can supply the demand and, as a consequence, while the flow reaching patient 4 remain steady at 6L/min, a deflation phase occurs. After about 20 min (t=1200s) the volume Vb3 in bag 230 becomes null (i.e., equals zero) and the delivery device 2 is no longer able to provide the steady supply of 6 L/min that does progressively decrease until the canister 10 is empty.

As described in Figure 4D the cap 225 can be set on its position "3" which decreases the opening pressure of membrane 220 to, for instance, 200mb. This in turn largely affects the performances of the Venturi system made of nozzle 202 and chamber 233. The flow going into nozzle 202 and exiting through outlet orifice 204 is lowered in this setting to about 0.75 L/min, which also decreases the suction capacity of said Venturi system (the amount of flow drawn from bag 230 to chamber 233) from 6 to 4-fold. This results in a total flow of about 3L/min in conduct 234, reaching patient 4 via breathing pathway 3.

More generally speaking, a gas delivery device 2 according to the present disclosure can be from about 5 to 10 times cheaper than a pressure regulator according to the prior art, and is able to deliver steady flows of 3 or 6 L/min for about 20 minutes that is sufficient for the first responders to arrive and take care of the patient.

Of course, higher flows can be obtained with the gas delivery device 2 according to the present disclosure, for instance 12 L/min in case of critical emergency such as cardiac arrest, by modifying the diameter of orifice 213, increasing the internal volume 101 of canister 10 and opening pressure of membrane 220.

Further, a gas cylinder equipped with a gas delivery device 2 according to the present invention constitutes a rather compact assembly that can be used in emergency vehicles, such as in ambulances or the like.

## Claims

1. Gas delivery device (2) comprising:
- a main chamber (201),
- an injection nozzle (202) comprising an inner gas passage (202a) in fluid communication with the main chamber (201), said inner gas passage (202a) comprising an inlet diameter (203) and an outlet diameter (204), said inlet diameter (203) being greater than the outlet diameter (204),
- a downstream chamber (233) in fluid communication with the inner gas passage (202a) of the injection nozzle (202),
- an inflatable bag (230) in fluid communication with the downstream chamber (233),
- a second chamber (221) fluidly communicating with the main chamber (201) and further with the inflatable bag (230), and
- a gas-flow control device (220, 224) arranged between the main chamber (201) and the second chamber (221) for controlling the passage of gas from the main chamber (201) to the second chamber (221),
**characterized in that**:
- the injection nozzle (202) is arranged downstream of said main chamber (201) so that the gas exiting the main chamber (201) enters into the inner gas passage (202a) of the injection nozzle (202), and
- the inflatable bag (230) comprises a bag inlet (231) and a bag outlet (232) so that:
• the second chamber (221) is fluidly connected to the bag inlet (231) of the inflatable bag (230), and
• the downstream chamber (233) is fluidly connected to the bag outlet (232) of the inflatable bag (230).

2. Gas delivery device (2) according to the preceding claim, **characterized in that** the inner gas passage (202a) has a conical or tronconical shape.

3. Gas delivery device (2) according to any one of the preceding claims, **characterized in that** the gas flow control device (220, 224) comprises a flexible membrane (220).

4. Gas delivery device (2) according to any one of the preceding claims, **characterized in that** the gas flow control device (220, 224) further comprises an elastic element cooperating with a flexible membrane (220).

5. Gas delivery device (2) according to any one of the preceding claims, **characterized in that** the inflatable bag (230) is in fluid communication with the second chamber (221) through a connection conduct (222).

6. Gas delivery device (2) according to claim 3, **characterized in that** the flexible membrane (220) is made of silicon.

7. Gas delivery device (2) according to claim 4, **characterized in that** the elastic element comprises a spring element.

8. Gas delivery device (2) according to any one of the preceding claims, **characterized in that** the inflatable bag (230) is made of a flexible material.

9. Gas delivery device (2) according to any one of the preceding claims, **characterized in that** the flexible inflatable bag (230) is made of polyethylene (PE) or a combination of polyethylene and one or several aluminum foils.

10. Gas cylinder comprising a gas delivery device (2) according to any one of the preceding claims, mounted on said gas cylinder.

11. Gas cylinder according to claim 10, **characterized in that** it is an oxygen cylinder or oxygen cartridge (10).

## Patentansprüche

1. Gaszufuhrvorrichtung (2), umfassend:
- eine Hauptkammer (201),
- eine Einspritzdüse (202), die einen inneren Gasdurchlass (202a) in Fluidverbindung mit der Hauptkammer (201) umfasst, wobei der innere Gasdurchlass (202a) einen Einlassdurchmesser (203) und einen Auslassdurchmesser (204) umfasst, wobei der Einlassdurchmesser (203) größer ist als der Auslassdurchmesser (204),
- eine nachgelagerte Kammer (233) in Fluidverbindung mit dem inneren Gasdurchlass (202a) der Einspritzdüse (202),
- einen aufblasbaren Beutel (230) in Fluidverbindung mit der nachgelagerten Kammer (233),
- eine zweite Kammer (221), die fluidisch mit der Hauptkammer (201) und ferner mit dem aufblasbaren Beutel (230) in Verbindung steht, und
- eine Gasflusssteuereinrichtung (220, 224), die zum Steuern des Durchlasses von Gas aus der Hauptkammer (201) in die zweite Kammer (221) zwischen der Hauptkammer (201) und der zweiten Kammer (221) angeordnet ist,
**dadurch gekennzeichnet, dass**:
- die Einspritzdüse (202) der Hauptkammer (201) nachgelagert angeordnet ist, sodass das aus der Hauptkammer (201) austretende Gas in den inneren Gasdurchlass (202a) der Einspritzdüse (202) eintritt, und
- der aufblasbare Beutel (230) einen Beuteleinlass (231) und einen Beutelauslass (232) umfasst, sodass:
• die zweite Kammer (221) fluidisch mit dem Beuteleinlass (231) des aufblasbaren Beutels (230) verbunden ist, und
• die nachgelagerte Kammer (233) fluidisch mit dem Beutelauslass (232) des aufblasbaren Beutels (230) verbunden ist.

2. Gaszufuhrvorrichtung (2) nach dem vorhergehenden Anspruch, **dadurch gekennzeichnet, dass** der innere Gasdurchlass (202a) eine konische oder tronkonische Form aufweist.

3. Gaszufuhrvorrichtung (2) nach einem der vorhergehenden Ansprüche, **dadurch gekennzeichnet, dass** die Gasflusssteuereinrichtung (220, 224) eine flexible Membran (220) umfasst.

4. Gaszufuhrvorrichtung (2) nach einem der vorhergehenden Ansprüche, **dadurch gekennzeichnet, dass** die Gasflusssteuereinrichtung (220, 224) ferner ein elastisches Element umfasst, das mit einer flexiblen Membran (220) zusammenwirkt.

5. Gaszufuhrvorrichtung (2) nach einem der vorhergehenden Ansprüche, **dadurch gekennzeichnet, dass** der aufblasbare Beutel (230) durch eine Verbindungsleitung (222) mit der zweiten Kammer (221) in Fluidverbindung steht.

6. Gaszufuhrvorrichtung (2) nach Anspruch 3, **dadurch gekennzeichnet, dass** die flexible Membran (220) aus Silizium besteht.

7. Gaszufuhrvorrichtung (2) nach Anspruch 4, **dadurch gekennzeichnet, dass** das elastische Element ein Federelement ist.

8. Gaszufuhrvorrichtung (2) nach einem der vorhergehenden Ansprüche, **dadurch gekennzeichnet, dass** der aufblasbare Beutel (230) aus einem flexiblen Material besteht.

9. Gaszufuhrvorrichtung (2) nach einem der vorhergehenden Ansprüche, **dadurch gekennzeichnet, dass** der aufblasbare Beutel (230) aus Polyethylen (PE) oder einer Kombination aus Polyethylen und einer oder mehreren Aluminiumfolien besteht.

10. Gaszylinder, umfassend eine Gaszufuhrvorrichtung (2) nach einem der vorhergehenden Ansprüche, die an dem Gaszylinder montiert ist.

11. Gaszylinder nach Anspruch 10, **dadurch gekennzeichnet, dass** es sich um einen Sauerstoffzylinder oder eine Sauerstoffpatrone (10) handelt.

## Revendications

1. Dispositif de distribution de gaz (2) comprenant :
- une chambre principale (201),
- une buse d'injection (202) comprenant un passage de gaz interne (202a) en communication fluidique avec la chambre principale (201), ledit passage de gaz interne (202a) comprenant un diamètre (203) d'orifice d'admission et un diamètre (204) d'orifice d'évacuation, ledit diamètre (203) d'orifice d'admission étant supérieur au diamètre (204) d'orifice d'évacuation,
- une chambre aval (233) en communication fluidique avec le passage de gaz interne (202a) de la buse d'injection (202),
- un sac gonflable (230) en communication fluidique avec la chambre aval (233),
- une seconde chambre (221) en communication fluidique avec la chambre principale (201) et en outre avec le sac gonflable (230), et
- un dispositif de régulation de flux gazeux (220, 224) disposé entre la chambre principale (201) et la seconde chambre (221) pour réguler le passage de gaz depuis la chambre principale (201) vers la seconde chambre (221),
**caractérisé en ce que** :
- la buse d'injection (202) est disposée en aval de ladite chambre principale (201) de sorte que le gaz quittant la chambre principale (201) entre dans le passage de gaz interne (202a) de la buse d'injection (202), et
- le sac gonflable (230) comprend un orifice d'admission (231) de sac et un orifice d'évacuation (232) de sac de sorte que :
• la seconde chambre (221) soit en communication fluidique avec l'orifice d'admission (231) de sac du sac gonflable (230), et
• la chambre aval (233) soit en communication fluidique avec l'orifice d'évacuation (232) de sac du sac gonflable (230).

2. Dispositif de distribution de gaz (2) selon la revendication précédente, **caractérisé en ce que** le passage de gaz interne (202a) a une forme conique ou tronconique.

3. Dispositif de distribution de gaz (2) selon l'une quelconque des revendications précédentes, **caractérisé en ce que** le dispositif de régulation de flux gazeux (220, 224) comprend une membrane souple (220).

4. Dispositif de distribution de gaz (2) selon l'une quelconque des revendications précédentes, **caractérisé en ce que** le dispositif de régulation de flux gazeux (220, 224) comprend en outre un élément élastique coopérant avec une membrane souple (220).

5. Dispositif de distribution de gaz (2) selon l'une quelconque des revendications précédentes, **caractérisé en ce que** le sac gonflable (230) est en communication fluidique avec la seconde chambre (221) par le biais d'une conduite de raccordement (222).

6. Dispositif de distribution de gaz (2) selon la revendication 3, **caractérisé en ce que** la membrane souple (220) est en silicium.

7. Dispositif de distribution de gaz (2) selon la revendication 4, **caractérisé en ce que** l'élément élastique comprend un élément à ressort.

8. Dispositif de distribution de gaz (2) selon l'une quelconque des revendications précédentes, **caractérisé en ce que** le sac gonflable (230) est en matériau souple.

9. Dispositif de distribution de gaz (2) selon l'une quelconque des revendications précédentes, **caractérisé en ce que** le sac gonflable souple (230) est en polyéthylène (PE) ou une combinaison de polyéthylène et d'une ou de plusieurs feuilles d'aluminium.

10. Bouteille de gaz comprenant un dispositif de distribution de gaz (2) selon l'une quelconque des revendications précédentes, monté sur ladite bouteille de gaz.

11. Bouteille de gaz selon la revendication 10, **caractérisée en ce qu'**il s'agit d'une bouteille d'oxygène ou d'une cartouche d'oxygène (10).
